# NEW EUROPEAN PATENT SPECIFICATION

(11) **EP 1 938 774 B2**
(45) Date of publication and mention of the opposition decision: **21.05.2014**
(45) Mention of the grant of the patent: 20.04.2011
(21) Application number: 05848160.7
(22) Date of filing: 31.12.2005
(51) Int. Cl.: A61K 38/01, A61F 2/04, A61L 27/22, A61L 27/40, A61L 27/54, A61L 29/04, A61L 29/14, A61K 35/64, A61L 27/48, A61L 31/00, A61L 31/04, A61L 31/12, A61L 27/36

(54) **MEDICAL ARTIFICIAL NERVE GRAFT CONTAINING SILK FIBROIN AND ITS PREPARATION METHOD**
MEDIZINISCHES KÜNSTLICHES NERVENTRANSPLANTAT MIT SEIDEN-FIBROIN UND SEIN HERSTELLUNGSVERFAHREN
GREFFON NERVEUX ARTIFICIEL À USAGE MÉDICAL CONTENANT DE LA FIBROÏNE DE SOIE ET PROCÉDÉ D'ÉLABORATION

(30) Priority: 28.09.2005 CN 200510094683
(43) Date of publication of application: 02.07.2008
(73) Proprietor: Nantong University, Nantong Jiangsu 226001 (CN)
(72) Inventor: GU, Xiaosong, Jiangsu 226001 (CN); YANG, Yumin, Jiangsu 226001 (CN); DING, Fei, Jiangsu 226001 (CN); ZHANG, Peiyun, Jiangsu 226001 (CN); LIU, Yan, Jiangsu 226001 (CN); LIU, Mei, Jiangsu 226001 (CN); CHEN, Xuemei, Jiangsu 226001 (CN); WANG, Xiaodong, Jiangsu 226001 (CN)
(74) Representative: Rippel, Hans Christoph
(86) International application number: PCT/CN2005/002423
(87) International publication number: WO 2007/036084

(56) References cited:
- WO-A2-01/25403
- WO-A2-2004/000915
- WO-A2-2004/001103
- WO-A2-2004/062697
- CN-A- 1 319 379
- CN-A- 1 372 023
- CN-A- 1 397 355
- CN-A- 1 475 533
- CN-A- 1 580 253
- GB-A- 2 386 841
- JP-A- 2004 208 808
- US-A1- 2003 100 108
- US-A1- 2004 005 363
- US-B1- 6 214 021
- ALDINI N N ET AL: "Effectiveness of a bioabsorbable conduit in the repair of peripheral nerves" BIOMATERIALS, ELSEVIER SCIENCE PUBLISHERS BV., BARKING, GB, vol. 17, no. 10, 1 May 1996 (1996-05-01), pages 959-962, XP004032679 ISSN: 0142-9612
- SHIN H ET AL: "Biomimetic materials for tissue engineering" BIOMATERIALS, ELSEVIER SCIENCE PUBLISHERS BV., BARKING, GB, vol. 24, no. 24, 1 November 2003 (2003-11-01), pages 4353-4364, XP004446079 ISSN: 0142-9612

## Description

### Field of the Invention

The present invention relates to a medical artificial nerve graft used in bridging of nerve damage and its preparation method.

### Background of the Invention

Clinically peripheral nerve coloboma resulting from trauma (or surgery) is seen more often. But when long-distant nerve coloboma cannot be made up by the method of end-to-end suture, it is necessary to use a graft in bridging. Although seeking and studying a more suitable graft has been going on for more than one hundred years, people still failed to find an ideal nerve graft which can be mostly recognized and widely used in clinic, except that auto-nerve has become the first choice of graft in bridging of nerve coloboma. The auto-nerve graft fails in extensive clinical application due to limited nerve graft source, difficult match of tissue structure and size, long-term denervation in the graft region.

The emergency and developing of tissue engineering provides a new path for constructing auto-nerve graft. In recent over 20 years, people have tried to use some biodegradable materials in peripheral nerve tissue engineering, usually some high polymers, including their copolymers and derivates of more than 10 kinds, such as polyglycolic acid (PGA), polylactic acid (PLA), chitosan, gelatin, silica gel, etc. (e.g. , China Patent CN1126515C, etc.). But they have different disadvantages in practical application.

US2003/0100108 A1 discloses a novel silk-fiber-based matrix having a wire-rope geometry for use in producing a ligament or tendon, particularly an anterior cruciate ligament, ex vivo for implantation into a recipient in need thereof. N. Nicoli et al. evaluated in an animal model a new conduit made with a bioabsorbable copolymer, poly(L-lactide-co-6-caprolactone), as a guide for nerve regeneration (Biomaterials 17, 1996, 959-962).

### Summary of the Invention

### Technology:

The objective of the present invention is to provide a medical artificial nerve graft containing high purity silk fibroin with good effect, good biocompatibility with the human body and excellent biodegradation properties, and its preparation method.

### Technic proposal:

The technic proposal of the present invention is as follows.

A medical artificial nerve graft containing high purity silk fibroin comprises vessel or comprises vessel and fiber scaffolds, wherein at least one of the vessel and the fiber scaffold contains a component of high purity silk fibroin.

The vessel contains a high purity silk fibroin component. The vessel also contains chitosan. The fiber scaffold is the high purity silk fibroin fiber.

A preparation method of a medical artificial nerve graft containing high purity silk fibroin, includes the following steps:
(1) Preparing high purity silk into a silk fibroin fiber;
(2) Preparing high purity silk fibroin fiber into a vessel containing high purity silk fibroin.

Chitosan is also added in step (2), and the prepared vessel containing high purity silk fibroin also contains chitosan.

The prepared vessel also combines with the fiber scaffold, i.e., inlaying the fiber scaffold into the vessel containing high purity silk fibroin. The fiber scaffold is the high purity silk fibroin fiber.

### Advantages:

Natural silk fibroin is a traditional feeding product in China. It is also the main species in fibroin fiber. The fibroin accounts for 70 per cent to 80 per cent of the silk weight.

It is the essential material that people use. The fibroin comprises 18 amino acids. Our experimental studies show it has good bioaffinity, non-toxicity, non-pollution, biogradation. The prepared artificial nerve graft has better effect. According to the literature retrieval, no artificial nerve graft prepared from silk fibroin has been found to treat nerve damage at home and abroad. The material of the product in the present invention is a high purity silk fibroin. It is natural degradable material and has good biocompatibility with human body. The prepared product contains no exogenous substance with toxic or side effect due to preparation technologies. The wall of the vessel has three dimensional structure provided with numerous micropores. It provides necessary path for nutrient transfer needed in the neurocyte growth process, with good effect obtained. It provides essential induction and necessary growth space for neurocyte growth.

We co-cultured the prepared product with nerve tissue cells *in vitro.* The product was found to have good histocompatibility after morphological observation, determination of acid metabolic activity, determination of nerve growth factor expression. The product was *in vivo* used to repair a 3-cm coloboma in rat sciatic nerve. It was found to be advantageous to the nerve regeneration, and the recovery of the damaged nerve function. Meanwhile, the product had good biocompatibility.

### Detailed Description of the Embodiments

The present invention is further illustrated with detailed embodiments as follows: Embodiment 1 (non-inventive):
(1) The preparation of high purity silk fibroin fiber (prepared according to routine methods or purchased from finished product of silk fibroin fiber) or preparation according to the following methods: Use the temperature of 50-100 degree Celsius for heat treatment of the silk in alkalescent solution (0.1-10percent sodium carbonate or 0.1-10percent potassium carbonate). Then wash the treated fiber in distilled water and obtain the high purity silk fibroin fiber, part of the fiber is used in step (2), while the other is used in step (3) as the fiber scaffold.
(2) The preparation of a high purity silk fibroin vessel: Dissolve part of the prepared silk fibroin fiber in calcium chloride-alcohol-water ternary solutions (the molar ratio of calcium chloride-alcohol-water is 1:2:8) at 25-80 degree Celsius (such as 25 degree Celsius, 50 degree Celsius, and 80 degree Celsius) for 0.5-6 h (such as 0.5h, 3h, and 6 h). Fill the dissolved mixture solution into cellulose membrane bags and dialyze it with distilled water. Inject the dialyzed fibroin solution into the mould, and conduct modeling process after drying. Then put the preliminarily mould fibroin vessel in the solution of methanol and sodium hydrate (1 mol/L) with 50:50 (V:V) for a certain time (5 min-2 h), then wash it with sodium hydrate (1 mol/L), 50 mmol/L phosphate buffer solution, distilled water in this order, and obtain the high purity silk fibroin fiber vessel.
(3) Combination: Inlay the obtained high purity silk fibroin fiber vessel into the vessel containing high purity silk fibroin, and combine the medical artificial nerve graft containing high purity silk fibroin.

### Embodiment 2 (non-inventive)

A preparation method of a medical artificial nerve graft containing high purity silk fibroin, includes the following steps:
(1) The preparation of a high purity silk fibroin vessel: Obtain the high purity silk fibroin vessel according to the same method as embodiment 1.
(2) Directly using the vessel containing high purity silk fibroin in nerve regeneration, or using it in nerve regeneration after filling an other substance into it; or inlaying the prepared high purity silk fibroin vessel into a fiber scaffold which is made by other materials (such as PGA, PLA, chitosan, chitin) then using it in nerve regeneration. See contents of China Patent CN1126515C for the fiber scaffold preparation methods by PGA, PLA, chitosan. The method for the fiber scaffold prepared from chitin is as follows: Dissolving chitosan in a weak acidic solution, such as acetic acid, or phosphoric acid, citric acid, lactic acid at a concentration of 2-15 percent (such as 2 percent, 8 percent, and 15 percent). Then processing the solution into the chitosan fiber using wet spinning to obtain the chitosan fiber. Putting the obtained chitosan fiber into the alcoholic solution containing acetic anhydride to have an acetylation reaction at 0-60 degree Celsius (such as 0 degree Celsius, 30 degree Celsius, and 60 degree Celsius) for 0.5-6 h (such as 0.5h, 2h, 4h, and 6 h). In the alcoholic solution containing acetic anhydride, the concentration of acetic anhydride is 1-50 per cent (such as 1 per cent, 20 per cent, 40 per cent, and 50 per cent). The alcoholic solution is methanol (or alcohol). Washing it with water after acetylation reaction, and putting it in 1 mol/L sodium hydrate or potassium hydrate for 24 h. Washing it with water and obtain the chitin fiber (i.e., fiber scaffold) after drying.

### Embodiment 3 (inventive)

A preparation method of a medical artificial nerve graft containing high purity silk fibroin, includes the following steps:
(1) The preparation of a high purity silk fibroin vessel: Dissolving the silk fibroin fiber obtained in the same method as in embodiment 1 in calcium chloride-alcohol-water ternary solutions (the molar ratio of calcium chloride-alcohol-water is 1:2:8) at 25-80 degree Celsius (such as 25 degree Celsius, 50 degree Celsius, and 80 degree Celsius) for 0.5-6 h (such as 0.5h, 2h, and 6h). Filling the dissolved mixture solutions into cellulose membrane bags, dialyzing it with the distilled water, and obtaining the silk fibroin solution.

Dissolving chitosan in a weak acidic solution such as acetic acid, or phosphoric acid, citric acid, lactic acid at a concentration of 2-15 percent (such as 2 percent, 8 percent , and 15 percent ). Then mixing up the chitosan solutions in the silk fibroin solution at certain ratio of weight (such as 1:2, 1:1, and 2: 1). Then injecting the aforementioned mixture solution in a mould, and conducting a modeling process after cryodesiccation. Then putting the preliminarily mould fibroin vessel into a solution of methanol and sodium hydrate(1 mol/L) with 50:50(V:V) for a certain time (5 min-2 h), then washing it with sodium hydrate (1 mol/L), 50 mmol/L phosphate buffer solution, and distilled water in this order, and obtaining the complex vessel containing silk fibroin. (2) Directly using the vessel containing high purity silk fibroin in nerve regeneration, or using it in nerve regeneration after filling another substance into it. Or inlaying the prepared silk fibroin vessel into the fiber scaffold which is made by other materials (such as PGA, PLA, chitosan, chitin) then using it in nerve regeneration.

### Embodiment 4 (non-inventive)

A preparation method of a medical artificial nerve graft containing high purity silk fibroin, includes the following steps:
(1) Obtaining the high purity silk fibroin fiber (i.e., fiber scaffold) according to the same method as embodiment 1.
(2) Inlaying the prepared fiber scaffold into the high purity silk fibroin vessel which is made by other materials (such as PGA, PLA, chitosan, chitin) then using it in nerve regeneration. See the contents of China Patent CN1126515C for the vessel preparation methods by PGA, PLA, chitosan. The method for the vessel prepared from chitin is as follows: Dissolving chitosan in a weak acidic solution, such as acetic acid, or phosphoric acid, citric acid, lactic acid at a concentration of 2-15 percent (such as 2percent, 8percent, and 15percent). Then letting the solution pass through a tubiform mould, and shaping in an alkaline coagulation solution such as a solution containing 8-20percent sodium hydrate (or 8-20percent potassium hydrate, or 8-20percent sodium carbonate, and which may also contain 5-30percent alcohol). Washing the vessel with water after moulding, and obtaining the chitosan vessel. Then putting the obtained chitosan vessel in an alcoholic solution containing acetic anhydride to have an acetylation reaction at 0-60 degree Celsius (such as 0 degree Celsius, 30degree Celsius and 60 degree Celsius) for 0.5-6 h (such as 0.5h, 2h, 4h, and 6 h). The reaction time and temperature may affect the acetylation degree of chitin. A longer reaction time and a higher reaction temperature affords chitin with a higher acetylation degree. Conversely, one gets chitin with a lower acetylation degree. A chitin with a different acetylation degree can be got according to necessity. The concentration of acetic anhydride in the alcoholic solution is 1-50percent (such as 1 percent, 20percent, 40percent, and 50percent). The alcoholic solution is methanol (or alcohol). Washing it with water after the acetylation reaction, and putting it in 1 mol/L sodium hydrate or potassium hydrate for 24 h. Washing it with water and obtaining the chitin fiber scaffold after drying.

## Claims

1. A medical artificial nerve graft containing high purity silk fibroin comprises vessel or comprises vessel and fiber scaffolds, wherein the vessel contains a high purity silk fibroin component and wherein the wall of the vessel has three dimensional structure provided with numerous micropores and wherein the vessel contains chitosan.

2. The preparation method of a medical artificial nerve graft containing high purity silk fibroin as claimed in claim 1, including the following steps:
(1) Preparing silk into a high purity silk fibroin fiber;
(2) Preparing the high purity silk fibroin fiber into a vessel containing high purity silk fibroin and wherein chitosan is also added in step (2), and the prepared vessel containing high purity silk fibroin also contains chitosan.

3. The preparation method of a medical artificial nerve graft containing high purity silk fibroin as claimed in claim 2, wherein the prepared vessel also combines with the fiber scaffold, i.e., inlaying the fiber scaffold into the vessel containing high purity silk fibroin and wherein the fiber scaffold is the high purity silk fibroin fiber.

## Patentansprüche

1. Medizinisches künstliches Nerventransplantat enthaltend hochreines Seidenfibroin, enthaltend Gefäße oder Gefäße und Fasergerüste, wobei das Gefäß eine hochreine Seidenfibroinkomponente enthält und die Wand des Gefäßes eine dreidimensionale Struktur hat und zahlreiche Mikroporen besitzt, und wobei das Gefäß Chitosan enthält.

2. Verfahren zur Herstellung eines medizinischen künstlichen Nerventransplantats enthaltend hochreines Seidenfibroin gemäß Anspruch 1, umfassend die folgenden Schritte:
(1) Herstellung von einer hochreinen Seidenfibroinfaser aus Seide;
(2) Herstellung eines Gefäßes enthaltend hochreines Seidenfibroin aus der hochreinen Seidenfibroinfaser, und
wobei in Schritt (2) außerdem Chitosan hinzugefügt wird, und das hergestellte Gefäß, enthaltend hochreines Seidenfibroin, außerdem Chitosan enthält.

3. Verfahren zur Herstellung eines medizinischen künstlichen Nerventransplantats enthaltend hochreines Seidenfibroin gemäß Anspruch 2, wobei das hergestellte Gefäß außerdem mit dem Fasergerüst kombiniert wird, das heißt, dass das Fasergerüst in das Gefäß enthaltend hochreines Seidenfibroin eingelegt wird, und wobei das Fasergerüst die hochreine Seidenfibroinfaser ist.

## Revendications

1. Greffe nerveuse artificielle médicale contenant de la fibroïne de soie de pureté élevée, comprenant un vaisseau ou comprenant un vaisseau et des supports fibreux, le vaisseau contenant un composant de fibroïne de soie de pureté élevée, la paroi du vaisseau possédant une structure en trois dimensions comportant de nombreux micropores et le vaisseau contenant du chitosane.

2. Procédé de préparation d'une greffe nerveuse artificielle médicale contenant de la fibroïne de soie de pureté élevée selon la revendication 1, comprenant les étapes :
(1) de préparation de la soie pour obtenir une fibre de fibroïne de soie de pureté élevée ;
(2) de préparation de la fibre de fibroïne de soie de pureté élevée en un vaisseau contenant de la fibroïne de soie de pureté élevée, du chitosane étant également ajouté à l'étape (2), et le vaisseau préparé contenant de la fibroïne de soie de pureté élevée contenant également du chitosane.

3. Procédé de préparation d'une greffe nerveuse artificielle médicale contenant de la fibroïne de soie de pureté élevée selon la revendication 2, dans lequel le vaisseau préparé se combine également avec le support fibreux, c'est-à-dire en incrustant le support fibreux dans le vaisseau contenant de la fibroïne de soie de pureté élevée, le support fibreux représentant la fibre de fibroïne de soie de pureté élevée.
